(19) Europäisches Patentamt
European Patent Office
Office européen des brevets

(11) **EP 4 245 778 A1**

(12) **EUROPEAN PATENT APPLICATION**
published in accordance with Art. 153(4) EPC

(43) Date of publication:
**20.09.2023  Bulletin 2023/38**

(21) Application number: **21891859.7**

(22) Date of filing: **09.11.2021**

(51) International Patent Classification (IPC):
*C08F 2/44* (2006.01)    *A41D 19/00* (2006.01)
*A41D 19/04* (2006.01)    *A61B 42/10* (2016.01)
*B29C 41/14* (2006.01)    *C08F 2/22* (2006.01)
*C08F 2/38* (2006.01)     *C08F 236/18* (2006.01)
*C08K 3/06* (2006.01)     *C08K 3/22* (2006.01)
*C08L 11/02* (2006.01)

(52) Cooperative Patent Classification (CPC):
**A41D 19/00; A41D 19/04; A61B 42/10;**
**B29C 41/14; C08F 2/22; C08F 2/38; C08F 2/44;**
**C08F 236/18; C08K 3/06; C08K 3/22; C08L 11/02**

(86) International application number:
**PCT/JP2021/041179**

(87) International publication number:
**WO 2022/102613 (19.05.2022 Gazette 2022/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.11.2020  JP 2020187617**

(71) Applicant: **Resonac Corporation**
**Tokyo 105-8518 (JP)**

(72) Inventors:
• **KANEKO, Shu**
  **Tokyo 105-8518 (JP)**
• **OGAWA, Masahiro**
  **Tokyo 105-8518 (JP)**
• **OGAWA, Noriko**
  **Tokyo 105-8518 (JP)**
• **SHIBUYA, Akira**
  **Tokyo 105-8518 (JP)**

(74) Representative: **Strehl Schübel-Hopf & Partner**
**Maximilianstrasse 54**
**80538 München (DE)**

(54) **CHLOROPRENE COPOLYMER LATEX AND METHOD FOR PRODUCING SAME**

(57)    The present invention relates to a chloroprene copolymer latex, a chloroprene copolymer latex composition, a chloroprene copolymer rubber molded article, a dip-molded product, and a method for producing a chloroprene copolymer latex. The method for producing a chloroprene copolymer latex has a step of emulsion polymerizing 2-chloro-1,3-butadiene (A-1) and 2-methyl-1,3-butadiene (A-2) in the presence of sulfur (A-3).

EP 4 245 778 A1

**Description**

Technical Field

The present invention relates to a latex including, as a main component, a copolymer of 2-chloro-1,3-butadiene (chloroprene) and 2-methyl-1,3-butadiene, a method for producing the same, and a molded article, particularly a dip-molded product, using a composition including the latex.

Background Art

Isoprene rubber (IR) and chloroprene rubber (CR) are synthetic rubber having flexibility equivalent to that of natural rubber. Thus, isoprene rubber or chloroprene rubber has been recently used, instead of natural rubber, in a material for a product obtained by dip-molding of a composition (dip-molded products), especially surgical glove, as a countermeasure against allergy. Although chloroprene rubber can be produced less expensively than isoprene rubber, chloroprene rubber problematically has low production efficiency because of, for example, requiring vulcanization treatment at a high temperature in order to achieve target strength. In addition, in chloroprene rubber, the stress relaxation on application of a stress (a phenomenon in which a stress decreases over time when a certain strain is given) is larger than in isoprene rubber. Thus gloves produced using chloroprene rubber problematically has low followability to the movement of hand fingers of a user to thereby lower tactile sensations.

Techniques for improving the flexibility of chloroprene rubber have been disclosed. For example, Patent Literature 1 states that a vulcanized rubber product having flexibility can be produced by mixing a chloroprene polymer having 70% by weight or more of 1% by weight toluene insoluble content and a chloroprene polymer having 10% by weight or less of 1% by weight toluene insoluble content. Patent Literature 2 discloses a technique related to a latex containing a copolymer of chloroprene and 2,3-dichloro-1,3-butadiene. As for the stress relaxation rate, Patent Literature 3 discloses styrene-butadiene rubber having a stress relaxation rate of 50 to 70% upon elapsed time of 6 minutes after removal of a tensile stress at 100% elongation.

Citation List

Patent Literature

Patent Literature 1: JP2019-143002A
Patent Literature 2: JP2019-044116A
Patent Literature 3: JP2001-131812A

Summary of Invention

Technical Problem

However, no chloroprene rubber is known which can provide desirable flexibility and stress relaxation properties when a vulcanized rubber product therefrom is used as gloves and can be vulcanized under mild conditions. For example, the techniques described in Patent Literatures 1 and 2 have insufficiently investigated stress relaxation and thus have a room for reducing the stress relaxation of molded articles in order to improve the tactile sensations of gloves produced as a vulcanized rubber product (molded article). The technique described in Patent Literature 3 problematically cannot provide rubber products having sufficient flexibility for surgical gloves.

An object of the present invention is to provide chloroprene copolymer latex for providing a molded article having desirable flexibility and stress relaxation properties when in use as gloves by a vulcanization treatment under mild conditions.

Solution to Problem

As a result of intensive studies to solve the above-described problems, the present inventors have found that a chloroprene copolymer latex obtained by emulsion polymerizing 2-chloro-1,3-butadiene (chloroprene) (A-1) and 2-methyl-1,3-butadiene (A-2) in the presence of sulfur (A-3) enables the above-described problems to be solved, having completed the present invention.

That is, the present invention relates to a chloroprene copolymer latex composition and a molded article and a dip molded product obtained by curing the composition, according to the following [1] to [14].

[1] A method for producing a chloroprene copolymer latex (A), including a step of emulsion polymerizing 2-chloro-1,3-butadiene (A-1) and 2-methyl-1,3-butadiene (A-2) in the presence of sulfur (A-3).

[2] The method for producing a chloroprene copolymer latex (A) according to [1], wherein a proportion of 2-methyl-1,3-butadiene (A-2) in the total monomer components used in the step of emulsion polymerization is 2 to 60 mol%, and a polymerization conversion of the total monomers is 61 to 90% by mass.

[3] The method for producing a chloroprene copolymer latex (A) according to [1] or [2], wherein an amount of the sulfur (A-3) to be used in the emulsion polymerization is 0.1 to 1.0 parts by mass per 100 parts by mass of the total amount of 2-chloro-1,3-butadiene (A-1) and 2-methyl-1,3-butadiene (A-2).

[4] A chloroprene copolymer latex (A), being a latex of a chloroprene copolymer, and
obtained by emulsion polymerizing 2-chloro-1,3-butadiene (A-1) and 2-methyl-1,3-butadiene (A-2) in the presence of sulfur (A-3).

[5] The chloroprene copolymer latex (A) according to [4], wherein a tetrahydrofuran insoluble content ratio of the chloroprene copolymer at 25°C is 5 to 80% by mass.

[6] The chloroprene copolymer latex (A) according to [4] or [5], wherein a tetrahydrofuran soluble component in the chloroprene copolymer at 25°C has a weight average molecular weight of 400,000 or more.

[7] The chloroprene copolymer latex (A) according to any one of [4] to [6], wherein an amount of the sulfur (A-3) to be used in the emulsion polymerization is 0.1 to 1.0 parts by mass per 100 parts by mass of the total amount of 2-chloro-1,3-butadiene (A-1) and 2-methyl-1,3-butadiene (A-2).

[8] The chloroprene copolymer latex (A) according to any one of [4] to [7], wherein a proportion of a monomer unit derived from 2-methyl-1,3-butadiene (A-2) is 10 to 50 mol% in the total monomer units constituting the chloroprene copolymer.

[9] A chloroprene copolymer latex composition, including 100 parts by mass in total of the chloroprene copolymer latex (A) according to any one of [4] to [8] and optionally a synthetic rubber (F), and

including 0.1 to 20.0 parts by mass of a metal oxide (B) ;
0.1 to 10.0 parts by mass of a vulcanization accelerator (C) ;
0.1 to 10.0 parts by mass of sulfur (D), and
0.1 to 10.0 parts by mass of an antioxidant (E).

[10] The chloroprene copolymer latex composition according to [9], wherein the composition includes a synthetic rubber(F), and
a proportion of the synthetic rubber (F) is 1 to 50% by mass per 100% by mass in total of solid content of the chloroprene copolymer latex (A) and the synthetic rubber (F).

[11] A molded article of a chloroprene copolymer rubber, obtained by curing the chloroprene copolymer latex composition according to [9] or [10].

[12] A dip-molded product obtained by molding the chloroprene copolymer latex composition according to [9] or [10] by a dipping method, followed by curing.

[13] The dip-molded product according to [12], wherein the dip-molded product is gloves.

[14] The dip-molded product according to [13], wherein the dip-molded product is medical disposable gloves.

Advantageous Effect of Invention

Provided is a chloroprene copolymer latex for providing a molded article having desirable flexibility and stress relaxation properties when in use as gloves by vulcanization treatment under mild conditions. That is, the chloroprene copolymer latex composition of the present invention can be vulcanized under mild conditions to provide a molded article (molded article of a chloroprene copolymer rubber) having small stress relaxation, having a high elastic modulus retention, and providing excellent tactile sensations. Use of sulfur (A-3) in the polymerization step of the chloroprene copolymer improves the tensile strength of a molded article, and thus the molded article according to the present invention can be suitably used for a dip-molded product, particularly for medical disposable gloves.

Description of Embodiment

Hereinafter, embodiments of the present invention will be described in detail, but the present invention is not limited to the configurations of the following embodiments. In the statements in claims and herein, "to" indicating a numerical range means not less than the lower limit value and not more than the upper limit value.

[Chloroprene copolymer latex (A)]

A chloroprene copolymer latex (A) can be obtained by emulsion polymerizing 2-chloro-1,3-butadiene (A-1) and 2-methyl-1,3-butadiene (A-2) in the presence of sulfur (A-3). In the chloroprene copolymer latex (A), particulates of a chloroprene copolymer produced in the emulsion polymerization step are dispersed in a dispersion medium such as water.

[Chloroprene copolymer]

The chloroprene copolymer included in the chloroprene copolymer latex (A) of the present invention includes at least structures (monomer units) derived from 2-chloro-1,3-butadiene (chloroprene) (A-1) and from 2-methyl-1,3-butadiene (A-2) .

The monomer units constituting the chloroprene copolymer may be only 2-chloro-1,3-butadiene (A-1) and 2-methyl-1,3-butadiene (A-2). The proportion of the monomer units derived from 2-chloro-1,3-butadiene (A-1) is preferably 50 to 90 mol%, more preferably 70 to 90 mol%, and further preferably 75 to 90 mol%, with respect to 100 mol% of the total monomer units constituting the chloroprene copolymer. The proportion of the monomer units derived from 2-methyl-1,3-butadiene (A-2) is preferably 10 to 50 mol%, more preferably 10 to 30 mol%, and further preferably 10 to 25 mol%, with respect to 100 mol% of the total monomer units constituting the chloroprene copolymer. As described below, the structures derived from sulfur are not counted as monomer units.

A proportion of the monomer units derived from 2-chloro-1,3-butadiene (A-1) in the chloroprene copolymer of 50 mol% or more is preferred because the polymerization reaction tends to progresses fast. A proportion of the monomer units derived from 2-chloro-1,3-butadiene (A-1) in the chloroprene copolymer of 90 mol% or less is preferred in respect that a molded article obtained by vulcanizing the chloroprene copolymer latex (A) has high flexibility.

When the proportion of the monomer units derived from 2-methyl-1,3-butadiene (A-2) in the chloroprene copolymer is 10 mol% or more, even vulcanizing a composition including the chloroprene copolymer under relatively mild conditions such as 110°C allows a sufficient strength to be developed in the resulting molded article. When the proportion of the monomer units derived from 2-methyl-1,3-butadiene (A-2) in the chloroprene copolymer is 50 mol% or less, a polymerization reaction is enabled to proceed relatively fast in an emulsion copolymerization reaction.

As the chloroprene copolymer is polymerized in the presence of the sulfur (A-3), at least a portion of the sulfur (A-3) coexistent with the monomers in polymerization is considered to be included in the chloroprene copolymer. The sulfur (A-3) is not included in a monomer (A-4) to be described below. It is presumed that the sulfur (A-3) serves as a chain transfer agent and does not become a monomer unit constituting the chloroprene copolymer.

The type of the sulfur (A-3) is not particularly limited. Powdered sulfur, precipitated sulfur, colloidal sulfur, surface-treated sulfur, insoluble sulfur, or the like can be used. The sulfur (A-3) may include an $S_8$ cyclic molecule or may include a linear molecule. One of the sulfurs (A-3) may be used singly, or two or more thereof may be used in combination. The sulfur (A-3) is preferably powdered sulfur.

As the sulfur (A-3) serves as a chain transfer agent, a larger amount of the sulfur (A-3) coexistent in emulsion polymerization tends to lead to a lower degree of polymerization of the chloroprene copolymer.

It is presumed that at least a portion of the sulfur (A-3) is not subjected to the chain transfer reaction and is included as is in the latex particles of the chloroprene copolymer to thereby form a crosslinked structure when the chloroprene copolymer is vulcanized. Accordingly, in comparison with a molded article provided from a latex including a chloroprene copolymer polymerized under conditions in which the sulfur (A-3) is absent, it is considered that the stress relaxation decreases and the tensile strength increases in a molded article provided from the chloroprene copolymer latex composition of the present invention. Sulfur atoms included in the sulfur (A-3) that has reacted as the chain transfer agent are presumed to be present, bonding to the chloroprene copolymer, but it is practically impossible to identify and structurally describe the state of individual bonds in the polymer including side chains. The structure of the chloroprene copolymer is presumed to vary depending on conditions such as the temperature and polymerization time in emulsion polymerization.

The structure provided when sulfur acts on the chloroprene copolymer is not particularly limited and is assumed to be -CH-SH-, -S-, -S-S-, -SC(=S)-, -SC(=S)O-, or the like. That is, a sulfur atom may be bonded to an end of the backbone or may form a crosslinked structure.

The amount of the sulfur (A-3) used in production of the chloroprene copolymer latex (A) is preferably 0.1 to 1.0 parts by mass, more preferably 0.1 to 0.8 parts by mass, and further preferably 0.1 to 0.5 parts by mass, per 100 parts by mass of the total amount of 2-chloro-1,3-butadiene (A-1) and 2-methyl-1,3-butadiene (A-2).

An amount of the sulfur (A-3) used in production of the chloroprene copolymer latex (A) of 0.1 parts by mass or more is preferred because the crosslinking reactivity of the chloroprene copolymer latex composition described below is excellent to thereby enable a molded article having excellent mechanical properties to be obtained by vulcanization treatment under conditions milder than before. An amount of the sulfur (A-3) of 1.0 part by mass or less is preferred because a decrease in the flexibility and yellowing of the molded article due to the sulfur (A-3) can be suppressed.

The tetrahydrofuran (THF) insoluble content ratio in the chloroprene copolymer at 25°C is usually 5 to 80% by mass, preferably 5 to 75% by mass, and more preferably 10 to 75% by mass. The tetrahydrofuran insoluble content is a gelled product of polymer chains via three-dimensional crosslinking in chloroprene copolymer particles. This tetrahydrofuran insoluble content ratio can be measured by a method employed in examples described below.

When the tetrahydrofuran insoluble content ratio in the chloroprene copolymer is 5% by mass or more, a molded article having relatively small stress relaxation can be provided, and thus tactile sensations of gloves produced from the chloroprene copolymer latex will be favorable. When the tetrahydrofuran insoluble content ratio in the chloroprene copolymer is 80% by mass or less, a molded article having excellent flexibility and tensile strength is provided. The tetrahydrofuran insoluble content ratio in the chloroprene copolymer can be controlled by adjusting the polymerization conversion, the amount of the sulfur (A-3), or the amount of the chain transfer agent used in production of the chloroprene copolymer (provided that the agent is not the sulfur (A-3)). The polymerization conversion can be controlled via the polymerization time and polymerization temperature of the chloroprene copolymer. A longer polymerization time tends to lead to a higher polymerization conversion, and a higher polymerization temperature tends to lead to a higher polymerization conversion.

For example, an increase in the polymerization conversion tends to increase the tetrahydrofuran insoluble content ratio in the chloroprene copolymer. An increase in the amount of the sulfur (A-3) coexistent in emulsion polymerization of the chloroprene copolymer tends to lower the tetrahydrofuran insoluble content ratio in the chloroprene copolymer. Further, an increase in the amount of the chain transfer agent tends to lower the tetrahydrofuran insoluble content ratio in the chloroprene copolymer.

The chloroprene copolymer can include monomer units derived from the monomer (A-4) as long as the object of the present invention is not impaired, in addition to the structures (monomer units) derived from 2-chloro-1,3-butadiene (A-1), structures (monomer units) derived from 2-methyl-1,3-butadiene (A-2), and structures (monomer units) derived from the sulfur (A-3). Here, the monomer (A-4) is a monomer other than 2-chloro-1,3-butadiene (A-1) or 2-methyl-1,3-butadiene (A-2), and is copolymerizable with at least one of 2-chloro-1,3-butadiene (A-1) and 2-methyl-1,3-butadiene (A-2). The monomer (A-4) may be a monomer copolymerizable with both 2-chloro-1,3-butadiene (A-1) and 2-methyl-1,3-butadiene (A-2). Examples of the monomer (A-4) include butadiene, 2,3-dichloro-1,3-butadiene, styrene, acrylonitrile, acrylic acid and esters thereof, and methacrylic acid and esters thereof. The chloroprene copolymer may include two types or more of the monomer (A-4).

The proportion (upper limit) of the monomer (A-4) when the chloroprene copolymer includes monomer (A-4) units is preferably 10 parts by mole or less, more preferably 8 parts by mole or less, and further preferably 5 parts by mole or less, with respect to 100 parts by mole in total of the monomer units derived from 2-chloro-1,3-butadiene (A-1) and the monomer units derived from 2-methyl-1,3-butadiene (A-2) in the chloroprene copolymer. The proportion (lower limit) of the monomer (A-4) when the chloroprene copolymer includes monomer (A-4) units is preferably 0.01 parts by mole or more, more preferably 0.1 parts by mole or more, and further preferably 0.5 parts by mole or more, with respect to 100 parts by mole in total of the monomer units derived from 2-chloro-1,3-butadiene (A-1) and the monomer units derived from 2-methyl-1,3-butadiene (A-2) in the chloroprene copolymer. When the proportion of the monomer (A-4) is 10 parts by mole or less with respect to 100 parts by mole in total of the monomer units derived from 2-chloro-1,3-butadiene (A-1) and the monomer units derived from 2-methyl-1,3-butadiene (A-2) in the chloroprene copolymer, the tensile strength and elongation of the molded article are favorable.

The weight average molecular weight of the tetrahydrofuran soluble component at 25°C of the chloroprene copolymer is preferably 400,000 or more, more preferably 500,000 or more, and further preferably 550,000, as measured by the method or conditions employed in examples described later. When the weight average molecular weight of the tetrahydrofuran soluble component at 25°C of the chloroprene copolymer is 400,000 or more, a molded article having favorable mechanical properties can be provided. The weight average molecular weight of the tetrahydrofuran soluble component at 25°C of the chloroprene copolymer is preferably 3,000,000 or less, more preferably 2,000,000 or less, and further preferably 900,000 or less. When the weight average molecular weight of the tetrahydrofuran soluble component at 25°C of the chloroprene copolymer is 3,000,000 or less, a molded article having favorable flexibility and tensile strength can be provided.

[Method for producing chloroprene copolymer latex (A)]

As a method for producing the chloroprene copolymer latex (A), a method of radically polymerizing 2-chloro-1,3-butadiene (A-1) and 2-methyl-1,3-butadiene (A-2) in an aqueous emulsion is simple and industrially advantageous.

Emulsion polymerizing 2-chloro-1,3-butadiene (A-1), 2-methyl-1,3-butadiene (A-2), and optionally the monomer (A-4) in the presence of the sulfur (A-3) in a dispersion medium such as water can provide a copolymer latex (A) including chloroprene copolymer particles dispersed in the dispersion medium such as water. The polymerization temperature on the emulsion polymerization is preferably 20 to 35°C, and the polymerization time is preferably 5 to 8 hours. The polymerization temperature and polymerization time on the emulsion polymerization are preferably within the above ranges because a desired polymerization conversion is achieved.

The content of 2-methyl-1,3-butadiene in the chloroprene copolymer can be adjusted by means of, for example, the proportions of 2-chloro-1,3-butadiene (A-1) and 2-methyl-1,3-butadiene (A-2) fed for polymerization and the polymerization conversion thereof.

A higher proportion of 2-methyl-1,3-butadiene (A-2) fed for polymerization with respect to the total monomers can finally result in a large content of the monomer units derived from 2-methyl-1,3-butadiene (A-2) with respect to the chloroprene copolymer. However, 2-methyl-1,3-butadiene (A-2) has lower reactivity at the beginning of the emulsion polymerization than that of 2-chloro-1,3-butadiene (A-1). Thus, a larger proportion of 2-methyl-1,3-butadiene fed tends to retard the progress of the polymerization to lengthen the reaction time.

As the polymerization of the chloroprene copolymer proceeds, 2-methyl-1,3-butadiene (A-2) is more likely to be incorporated in the polymer. Then, an increase in the polymerization conversion on polymerization for the chloroprene copolymer can lead to an increase in the content of 2-methyl-1,3-butadiene (A-2) with respect to the final chloroprene copolymer. With a low polymerization conversion, remaining monomers increase, which requires an effort of removing the remaining monomers, and moreover, mechanical properties of the molded article are degraded.

In view of the above, 2-methyl-1,3-butadiene (A-2) in the total monomer components used in polymerization is preferably 2 to 60 mol%, more preferably 10 to 50 mol%, and further preferably 15 to 35 mol%, in view of effectively providing the chloroprene copolymer in the present invention. The polymerization conversion of the total monomers is preferably 61% by mass or more, more preferably 75% by mass or more, and further preferably 80% by mass or more. The polymerization conversion of the total monomers is preferably 90% by mass or less and more preferably 85% by mass or less. When the polymerization conversion of the total monomers is 90% by mass or less, the quality of the chloroprene copolymer obtained by the polymerization is favorable, and the physical properties of a molded article provided from the chloroprene copolymer latex (A) are also favorable.

The emulsifier for the emulsion polymerization is preferably an anionic surfactant. Examples of the anionic surfactant include rosin acid soap, sodium salts of naphthalenesulfonic acid condensates, sodium salts of dodecylbenzenesulfonic acid, and sodium salts of dodecylsulfuric acid. Usual rosin acid soap can be used in view of simple operation for solidification. Particularly in view of coloring stability, a sodium salt and/or potassium salt of disproportionated rosin acid can be used. In view of stability of the particles in the latex, a salt of rosin acid soap and a dimer acid may be used.

The amount of the emulsifier used is preferably 0.5 to 20.0 parts by mass, more preferably 1.0 to 10.0 parts by mass, and further preferably 1.5 to 5.0 parts by mass, per 100 parts by mass in total of 2-chloro-1,3-butadiene (A-1), 2-methyl-1,3-butadiene (A-2), and the monomer (A-4). When the amount of the emulsifier used is 0.5 parts by mass or more, poor emulsification is unlikely to occur, and exotherm due to the polymerization can be controlled. When the amount of the emulsifier used is 0.5 parts by mass or more, problems do not arise, such as generation of aggregates and poor appearance of products. On the other hand, when the amount of the emulsifier used is 20.0 parts by mass or less, the emulsifier such as rosin acid does not remain in the chloroprene copolymer, and adhesion is unlikely to occur in the chloroprene copolymer. Thus, when the amount of the emulsifier used is 20.0 parts by mass or less, problems of processability and handleability due to, for example, adhesion of the chloroprene copolymer latex composition to the mold (former) on molding or adhesion of a molded article on use does not occur, and the color tone of the molded article does not deteriorate.

As a polymerization initiator, a usual radical polymerization initiator can be used. For example, an organic or inorganic peroxide such as benzoyl peroxide, potassium peroxide, ammonium persulfate, cumene hydroperoxide, and t-butyl hydroperoxide, or an azo compound such as azobisisobutyronitrile is used in the case of emulsion polymerization. One of the polymerization initiators may be used singly, or two or more thereof may be used in combination.

In polymerization of the chloroprene copolymer, a chain transfer agent (provided that the agent is not the sulfur (A-3)) can be used for adjusting the tetrahydrofuran insoluble content ratio. The amount of the chain transfer agent used is preferably 0.01 to 15.0 parts by mass, more preferably 0.05 to 10.0 parts by mass, and further preferably 0.1 to 1.0 parts by mass, per 100 parts by mass in total of 2-chloro-1,3-butadiene (A-1), 2-methyl-1,3-butadiene (A-2), and the monomer (A-4).

The chain transfer agent is not particularly limited, and a known chain transfer agent can be used, including an alkylmercaptan such as n-dodecylmercaptan, n-decylmercaptan, octylmercaptan, or tert-dodecylmercaptan, a dialkyl xanthogen disulfide such as diisopropyl xanthogen disulfide or diethyl xanthogen disulfide, or iodoform. More preferred is an alkylmercaptan, and further preferred is n-dodecylmercaptan.

In polymerization of the chloroprene copolymer, a cocatalyst may be used with the polymerization initiator, if desired. The cocatalyst that can be used with the polymerization initiator is not particularly limited, and a common cocatalyst can be used. Examples of the cocatalyst include anthraquinonesulfonates, potassium sulfite, sodium disulfite, sodium sulfite, tetraethylenepentamine, N,N-dimethyl-p-toluidine, and copper sulfate. One of the cocatalysts may be used singly, or two or more thereof may be used in combination.

Generally in emulsion polymerization, a polymerization terminator is added when a predetermined polymerization conversion is reached to thereby stop the polymerization reaction, in order to provide a polymer having a desired molecular weight and a desired molecular weight distribution. A polymerization terminator may be used also in the embodiment of the present invention. The type of polymerization terminator is not particularly limited, and a polymerization terminator usually used can be used, including phenothiazine, para-t-butylcatechol, hydroquinone, hydroquinone monomethylether, and diethylhydroxylamine. One of the polymerization terminators may be used singly, or two or more thereof may be used in combination.

In addition, a stabilizer such as an acid acceptor and/or an antioxidant may be blended to the chloroprene copolymer latex (A) as long as the object of the present invention is not impaired.

In addition, a thickener may be added to the chloroprene copolymer latex (A) as long as the object of the present invention is not impaired. The thickener that can be used is not particularly limited, and a common thickener can be used. For example, methylcellulose, a urethane-modified polyether, an acrylic polymer, or the like can be used. One of the thickeners may be used singly, or two or more thereof may be used in combination.

[Chloroprene copolymer latex composition]

The chloroprene copolymer latex composition includes the solid content of the chloroprene copolymer latex (A) obtained by the above polymerization method, a metal oxide (B), a vulcanization accelerator (C), sulfur (D), and an antioxidant (E). The solid content of the chloroprene copolymer latex (A) here refers to a component provided when allowing the chloroprene copolymer latex (A) to stand in an oven at 141°C for 30 minutes for drying. The component is obtained by removing water or the like, which is the dispersion medium, from the chloroprene copolymer latex (A). The chloroprene copolymer latex composition may include a dispersion medium such as water in the chloroprene copolymer latex (A).

When not including a synthetic rubber (F) described below, the chloroprene copolymer latex composition further includes 0.1 to 20.0 parts by mass of the metal oxide (B), 0.1 to 10.0 parts by mass of the vulcanization accelerator (C), 0.1 to 10.0 parts by mass of the sulfur (D), and 0.1 to 10.0 parts by mass of the antioxidant (E), per 100 parts by mass of the solid content of the chloroprene copolymer latex (A). Blending in this composition provides a rubber molded article (e.g., a film) having improved stress relaxation resistance against pulling from the chloroprene copolymer latex composition. Among the materials used for blending, a water-insoluble component and a component that destabilizes the colloid state of the chloroprene copolymer latex are each made into an aqueous dispersion in advance, and then the aqueous dispersion is added to the chloroprene copolymer latex.

The type of the metal oxide (B) is not particularly limited. Examples thereof that can be used include zinc oxide, lead oxide, and trilead tetraoxide, and zinc oxide is particularly preferred. One of the metal oxides (B) may be used singly, or two or more thereof may be used in combination.

The amount of the metal oxide (B) contained in the chloroprene copolymer latex composition according to the present embodiment is usually 0.1 to 20.0 parts by mass, preferably 0.5 to 15.0 parts by mass, and more preferably 0.5 to 10.0 parts by mass, per 100 parts by mass of the solid content of the chloroprene copolymer latex (A). When the amount of the metal oxide (B) is 0.1 parts by mass or more, the chloroprene copolymer can be cured by the vulcanization treatment, and when the amount thereof is 0.5 parts by mass or more, the chloroprene copolymer can be vulcanized more efficiently. When the amount of the metal oxide (B) is 20.0 parts by mass or less, a favorable crosslinked structure is obtained by the vulcanization treatment, and scorching is unlikely to occur. The colloid state of the chloroprene copolymer latex composition is stabilized, and thus, problems such as precipitation are unlikely to arise.

The type of the vulcanization accelerator (C) is not particularly limited, and it is possible to use a vulcanization accelerator commonly used for vulcanization treatment of an isoprene-based polymer latex or a chloroprene-based polymer latex. Examples thereof include thiuram-based, dithiocarbamate-based, thiourea-based, guanidine-based, and thiazole-based vulcanization accelerators.

Examples of the thiuram-based vulcanization accelerator include tetraethylthiuram disulfide and tetrabutylthiuram disulfide. Examples of the dithiocarbamate-based vulcanization accelerator include sodium dibutyldithiocarbamate, zinc dibutyldithiocarbamate, and zinc diethylthiodicarbamate. Examples of the thiourea-based vulcanization accelerator include ethylene thiourea, diethyl thiourea, trimethyl thiourea, and N,N'-diphenyl thiourea (DPTU). Examples of the guanidine-based vulcanization accelerator include diphenyl guanidine (DPG) and diorthotoluyl guanidine. Examples of the thiazole-based vulcanization accelerator include 2-mercaptobenzothiazole, di-2-benzothiazolyl disulfide, and zinc 2-mercaptobenzothiazole. One of the vulcanization accelerators (C) may be used singly, or two or more thereof may be used in combination.

The amount of the vulcanization accelerator (C) contained in the chloroprene copolymer latex composition according to the present embodiment is usually 0.1 to 10.0 parts by mass, preferably 0.5 to 5.0 parts by mass, and more preferably 0.5 to 3.0 parts by mass, per 100 parts by mass of the solid content of the chloroprene copolymer latex (A). When the amount of the vulcanization accelerator (C) is within this range, a moderate vulcanization rate can be achieved, lack of crosslinked structures due to insufficient vulcanization treatment is unlikely to occur, and additionally, scorching is unlikely to occur. Also, when the amount of the vulcanization accelerator (C) is set within the above range, a molded article provided from the chloroprene copolymer latex composition according to the present embodiment has a moderate vulcanization density (the density of the crosslinked structures obtained by the vulcanization treatment), and thus mechanical properties can be imparted to the molded article.

The sulfur (D), unlike the sulfur (A-3), is added to the chloroprene copolymer latex (A) in production of the chloroprene copolymer latex composition after the emulsion polymerization. The type of the sulfur (D) is not particularly limited. Powdered sulfur, precipitated sulfur, colloidal sulfur, surface-treated sulfur, and insoluble sulfur, as well as sulfur-containing compounds such as polysulfides and polymeric polysulfides (except for the above vulcanization accelerators) can be used. One of the sulfurs (D) may be used singly, or two or more thereof may be used in combination.

The amount of the sulfur (D) contained in the chloroprene copolymer latex composition according to the present embodiment is usually 0.1 to 10.0 parts by mass, preferably 0.2 to 7.0 parts by mass, and more preferably 0.8 to 5.0 parts by mass, per 100 parts by mass of the solid content of the chloroprene copolymer latex (A). When the amount of the sulfur (D) is within this range, a moderate vulcanization rate can be achieved, lack of crosslinked structures due to insufficient vulcanization treatment is unlikely to occur, and additionally, scorching is unlikely to occur. The colloid state of the chloroprene copolymer latex composition is stabilized, and thus, problems such as precipitation are unlikely to occur.

The type of the antioxidant (E) is not particularly limited. When a molded article having high heat resistance is desirable, an antioxidant that prevents thermal aging and an antioxidant that prevents ozone aging are preferably used in combination.

Examples of the antioxidant that prevents thermal aging include diphenylamine-based antioxidants such as octylated diphenylamine, p-(p-toluene-sulfonylamide) diphenylamine, and 4,4'-bis($\alpha,\alpha$-dimethylbenzyl) diphenylamine. Such an antioxidant imparts heat resistance to the molded article and can further impart stain resistance (such as suppression of discoloration) thereto.

Examples of the antioxidant that prevents ozone aging include N,N'-diphenyl-p-phenylenediamene (DPPD) and N-isopropyl-N'-phenyl-p-phenylenediamene (IPPD).

When the molded article of a chloroprene copolymer rubber according to the present embodiment is used as medical disposable gloves, appearances (in particular, color tone) and hygiene are considered important. Thus, as the antioxidant (E), a hindered phenolic antioxidant is preferably used. Examples of the hindered phenolic antioxidant include 2,2'-methylenebis-(4-ethyl-6-t-butylphenol) and 4,4'-methylenebis-(2,6-di-t-butylphenol).

The amount of the antioxidant (E) contained in the chloroprene copolymer latex composition according to the present embodiment is usually 0.1 to 10.0 parts by mass, preferably 0.5 to 5.5 parts by mass, and more preferably 2.0 to 4.8 parts by mass, per 100 parts by mass of the solid content of the chloroprene copolymer latex (A). When the amount of the antioxidant (E) is within this range, a sufficient antioxidant effect is provided, the vulcanization treatment is not inhibited, and additionally, the color tone is unlikely to deteriorate.

The chloroprene copolymer latex composition can include synthetic rubber (F) that can be mixed with the chloroprene copolymer latex (A). Incorporation of the synthetic rubber (F) in the chloroprene copolymer latex composition is preferred because rubber properties other than those of the chloroprene copolymer can be imparted to a molded article. The synthetic rubber (F) that can be mixed may be selected from isoprene rubber, chloroprene rubber (except for the chloroprene copolymer included in the chloroprene copolymer latex (A)), acrylonitrile-butadiene rubber, butadiene rubber, and the like. In view of the compatibility with the chloroprene copolymer latex (A), isoprene rubber and chloroprene rubber are preferred as the synthetic rubber (F). Two types or more of the synthetic rubber (F) may be used in the chloroprene copolymer latex composition as required.

The synthetic rubber (F) in the chloroprene copolymer latex composition may be blended as long as the object of the present invention is not impaired. When the chloroprene copolymer latex composition includes the synthetic rubber (F) that can be mixed, the proportion (upper limit) of the synthetic rubber (F) is preferably 50% by mass or less, more preferably 25% by mass or less, and further preferably 10% by mass or less, per 100 parts by mass in total of the solid content of the chloroprene copolymer latex (A) and the synthetic rubber (F). When the chloroprene copolymer latex composition includes the synthetic rubber (F), the lower limit of the proportion of the synthetic rubber (F) is preferably 1% by mass or more, more preferably 3% by mass or more, and further preferably 5% by mass or more. When the proportion of the synthetic rubber (F) is 50% by mass or less, the maturing time and vulcanization time of the chloroprene copolymer latex composition are not prolonged. A proportion of the synthetic rubber (F) of 1% by mass or more is preferred for allowing the characteristics of another synthetic rubber (F) to be developed.

The amount of the synthetic rubber (F) blended in the chloroprene copolymer latex composition is preferably 100 parts by mass or less, more preferably 33 parts by mass or less, and further preferably 10 parts by mass or less, per 100 parts by mass of the solid content of the chloroprene copolymer latex (A). The amount of the synthetic rubber (F) blended is preferably 1 part by mass or more, more preferably 3.1 parts by mass or more, and further preferably 5.3 parts by mass or more, per 100 parts by mass of the solid content of the chloroprene copolymer latex (A).

When the chloroprene copolymer latex composition includes the chloroprene copolymer latex (A) and the synthetic rubber (F), the chloroprene copolymer latex composition may include 0.1 to 20.0 parts by mass of metal oxide (B), 0.1 to 10.0 parts by mass of the vulcanization accelerator (C), 0.1 to 10.0 parts by mass of the sulfur (D), and 0.1 to 10.0 parts by mass of the antioxidant (E), per 100 parts by mass in total of the solid content of the chloroprene copolymer latex (A) and the synthetic rubber (F).

The synthetic rubber (F) may be a latex including particulates of the synthetic rubber (F) dispersed therein. When the latex of the synthetic rubber (F) is used, the chloroprene copolymer latex composition may include 0.1 to 20.0 parts by mass of the metal oxide (B), 0.1 to 10.0 parts by mass of the vulcanization accelerator (C), 0.1 to 10.0 parts by mass of the sulfur (D), and 0.1 to 10.0 parts by mass of the antioxidant (E), per 100 parts by mass in total of the solid content of the chloroprene copolymer latex (A) and the solid content of the latex of the synthetic rubber (F) .

To the chloroprene copolymer latex composition according to the present embodiment, other additives may be blended, if desired, in addition to the chloroprene copolymer latex (A), the metal oxide (B), the vulcanization accelerator (C), the sulfur (D), the antioxidant (E), and the synthetic rubber (F), as long as the object of the present invention is not impaired. Examples of the additives that can be blended include a pH adjuster, a filler, a pigment, a colorant, an antifoaming agent, and a thickener.

[Molded article of chloroprene copolymer rubber]

The chloroprene copolymer latex composition according to the present invention can be molded and cured to thereby provide a chloroprene copolymer rubber molded article. For example, the chloroprene copolymer latex composition according to the embodiment can be molded by a dip processing method to thereby provide a dip-molded product.

The chloroprene copolymer latex composition according to the present embodiment may be matured under predetermined conditions before the dip processing. The temperature conditions for the maturing is 15 to 40°C, and the maturing time is 15 to 72 hours. For example, conditions of maturing at 23°C for 20 hours may be employed. The starting point of the maturing is the time point when the chloroprene copolymer latex (A) is mixed with all of the metal oxide (B), the vulcanization accelerator (C), the sulfur (D), and the antioxidant (E).

After the maturing, the steps of a dip and solidification treatment, drying, and vulcanization treatment (curing) are conducted in this order to thereby provide a molded article in a film form.

The dip and solidification treatment can be conducted by submerging a plate or mold coated with a coagulant in the chloroprene copolymer latex composition for a predetermined time to thereby deposit the solid content in the chloroprene copolymer latex composition, including the chloroprene copolymer, on the surface of the plate or mold. This is presumed as follows: particulates coated with a film of an emulsifier or the like having surface activity have been formed in the chloroprene copolymer latex (A). The film of the particulates is disintegrated by the action of a coagulant adhering to the surface of the plate or mold, whereby the chloroprene copolymer and the like in the particulates adhere to the surface of the plate or mold. As the coagulant, a metal salt can be used. For example, a metal nitrate can be used.

In order to avoid the problem of the appearance of the molded article, such as generation of a blister or pinhole, a drying step at a relatively low temperature of 70°C or more and 100°C or less (roughly drying step) may be previously conducted before the vulcanization treatment.

The vulcanization temperature in the vulcanization treatment can be 110 to 140°C. The solid content of the chloroprene copolymer latex deposited by the dip and solidification treatment can be vulcanized at 110°C in air, for example. The vulcanization time in the above vulcanization temperature range can be 15 minutes or more and 90 minutes or less, for example. Sufficient vulcanization treatment is preferably conducted to the extent that the tensile strength and tensile elongation ratio of the molded article do not deteriorate.

Vulcanizing the composition deposited on the surface of the plate or mold under the above conditions can provide a molded article of a chloroprene copolymer rubber.

When the vulcanization treatment is conducted, a crosslinked structure is presumed to be formed, ascribed to both the sulfur (A-3) and the sulfur (D) remaining unreacted in polymerization, in the chloroprene copolymer deposited on the surface of the plate or mold. Here, the sulfur (A-3) has been included in the particulates before disintegration of the particulates, and thus the sulfur (A-3) tends to be easily deposited among the chloroprene copolymers included in the same particulate. As a result, it is presumed that formation of the crosslinked structure among the chloroprene copolymers included in the same particulate is more likely to be prompted by the sulfur (A-3). On the other hand, the sulfur (D) has been blended in the stage of producing the chloroprene copolymer latex composition and does not penetrate in the particulates of the chloroprene copolymer. Thus, the sulfur (D) tends to be easily deposited among the chloroprene copolymers present among different particulates before the disintegration of the particulates. As a result, it is presumed that formation of a crosslinked structure among molecules of the chloroprene copolymer present among different particulates before the disintegration of the particulates is more likely to be facilitated by the sulfur (D). Depending on the structure derived from the sulfur (A-3) or the status of the distribution of the sulfur (D), the crosslinked structure may be formed within one chloroprene polymer molecule. Crosslinked structures to be formed by the sulfur (A-3) and the sulfur (D) are presumed to be often formed particularly in a location at which monomer units derived from 2-methyl-1,3-butadiene (A-2) of the chloroprene polymer molecules are present.

As described above, when the chloroprene copolymer latex composition according to the present invention is vulcanized, crosslinked structures among the chloroprene copolymers included in the same particulate in the chloroprene copolymer latex (A) are presumed to be formed in a larger number than in a case where a chloroprene copolymer produced without use of the sulfur (A-3) is crosslinked. As a result, when the chloroprene copolymer latex composition according to the present invention is vulcanized, it is conceived that a chloroprene copolymer rubber molded article having a high tensile strength, excellent flexibility, and further small stress relaxation can be provided. In other words, subjecting the chloroprene copolymer latex composition according to the present invention to dip and solidification treatment is presumed to enable sulfur blended as the sulfur (A-3) to be dispersed in the solid content deposited on the surface of the plate or mold because unreacted sulfur (A-3) is included in the particulates of the chloroprene copolymer latex. As a result, when the sulfur (A-3) is used in production of the chloroprene copolymer latex, in comparison with a case in which the sulfur (A-3) is not used, it is conceived that the sulfur is finely distributed in the solid content deposited to thereby provide a molded body that is more flexible and has smaller stress relaxation than conventional molded articles.

In examples described below, a 100% elastic modulus is used as an index for the flexibility. A smaller 100% elastic modulus value indicates higher flexibility. A 100% elastic modulus retention is used as an index for the stress relaxation. A 100% elastic modulus retention value closer to 100% indicates smaller stress relaxation.

[Medical disposable gloves]

The molded article of a chloroprene copolymer rubber can be suitably used particularly as medical disposable gloves. The 100% elastic modulus, 500% elastic modulus, tensile strength, 100% elastic modulus retention, and tensile elongation ratio of the chloroprene copolymer rubber molded article can be measured by methods employed in examples described below.

A chloroprene copolymer rubber molded article having a 100% elastic modulus of 1.1 MPa or less has sufficient flexibility for medical disposable gloves. Regarding the lower limit, the 100% elastic modulus of the molded article of a chloroprene copolymer rubber may be 0.6 MPa or more, for example.

When the chloroprene copolymer rubber molded article has a 500% elastic modulus of 1.0 to 2.5MPa, the medical disposable gloves have a soft feeling of use, and a user is unlikely to be fatigued even if used for a long period.

The molded article of a chloroprene copolymer rubber preferably has a tensile strength of 20 MPa or more because breaks of the medical disposable gloves are unlikely to occur. Regarding the upper limit of the tensile strength of the molded article of a chloroprene copolymer rubber may be 35 MPa or less, for example.

When the 100% elastic modulus retention is 90% or more, the gloves has high followability to extension and contraction generated by motions of the hands of a user, and the user can have an excellent feeling of use. The upper limit of the 100% elastic modulus retention may be 95% or less, for example.

The molded article of a chloroprene copolymer rubber preferably has a tensile elongation ratio of 800% or more because breaks of the medical disposable gloves are unlikely to occur. Regarding the upper limit, the tensile elongation ratio of the molded article of a chloroprene copolymer rubber may be 1500% or less, for example.

Examples

Hereinafter, the present invention will be further described in detail with reference to examples, but the present invention is not intended to be limited to these examples.

<Method for calculating polymerization conversion>

The emulsion of the chloroprene copolymer was collected after the start of the polymerization, and the collected emulsion was allowed to stand in an oven at 141°C for 30 minutes for drying to thereby provide a dried solid substance. The dried solid substance obtained by the drying treatment included a polymer and solid content other than the polymer. Then, the mass of the component that did not evaporate at 141°C among the various components used for the emulsion polymerization was calculated from the amount of the polymerization material fed, and was used as the mass of the solid content other than the polymer. A value obtained by subtracting the mass of the solid content other than the polymer from the mass of the dried solid substance obtained by drying the emulsion after the start of the polymerization was used as the "amount of the chloroprene copolymer produced," and the polymerization conversion was calculated by the expression (1). The polymerization conversion calculated is shown in Table 1.

$$\text{Polymerization conversion [\% by mass]} = [(\text{amount of chloroprene copolymer produced})/(\text{mass of total monomers fed})] \times 100 \cdots (1)$$

The "mass of total monomers fed" in the expression (1) is the sum of the amount of 2-chloro-1,3-butadiene (A-1) fed, the amount of 2-methyl-1,3-butadiene (A-2) fed, and optionally the amount of the monomer (A-4) fed included in the emulsion of the amount collected for providing the dried solid substance.

[Method for measuring physical properties of chloroprene copolymer latex (A)]

The various physical properties of the chloroprene copolymer latex (A) provided were evaluated by the following methods.

<Tetrahydrofuran insoluble content ratio of chloroprene copolymer>

The tetrahydrofuran insoluble content ratio of the chloroprene copolymer was measured as follows. Specifically, at 25°C, 1 g of chloroprene copolymer latex (A) was added dropwise to 100 mL of tetrahydrofuran and shaken on a shaker (SA300) manufactured by Yamato Scientific Co., Ltd. for 10 hours. The mixture of the chloroprene copolymer latex (A) and tetrahydrofuran after the shaking treatment was subjected to separation by centrifugal sedimentation using a centrifugal sedimentation separator (manufactured by KOKUSAN Co. Ltd., H-9R) to provide a dissolution phase as a supernatant. The dissolution phase provided was heated to 100°C to evaporate the tetrahydrofuran over an hour, and the mass of the dried solid substance was measured. This provides the mass of the dissolved matters that were dissolved in the dissolution phase out of the chloroprene copolymer.

The mass of the chloroprene copolymer in 1 g of the chloroprene copolymer latex (A) and the mass of the above dissolved matters were substituted into the expression (2) to calculate the content of the tetrahydrofuran insoluble content that did not dissolve in tetrahydrofuran at 25°C (tetrahydrofuran insoluble content ratio) out of the chloroprene copolymer. The tetrahydrofuran insoluble content ratio is shown in Table 1.

$$\text{The tetrahydrofuran insoluble content ratio (\% by mass)} = \{1 - [(\text{mass of dissolved matters})/(\text{mass of chloroprene copolymer in 1 g of chloroprene copolymer latex (A)})]\} \times 100 \cdots (2)$$

The mass of the chloroprene copolymer in 1 g of the chloroprene copolymer latex (A) in the expression (2) was considered as the mass of the solid content obtained by drying 1 g of the chloroprene copolymer latex (A) to solid. The chloroprene copolymer latex (A), when dried to solid, was allowed to stand in an oven at 141°C for 30 minutes for drying.

<Weight average molecular weight (Mw)>

An exemplary method for determining the weight average molecular weight (Mw) of the tetrahydrofuran soluble component at 25°C in the chloroprene copolymer will be described below. In the same processing as for the sample preparation for the measurement of tetrahydrofuran insoluble content ratio described above, a dissolution phase as a supernatant after separation by centrifugal sedimentation was prepared, separated, and diluted with tetrahydrofuran to prepare a sample. The sample provided was subjected to molecular weight measurement in terms of polystyrene by GPC (gel permeation chromatography method) to measure the weight average molecular weight (Mw).

As for the GPC measurement conditions, LC-20AD manufactured by Shimadzu Corporation as a GPC measurement apparatus and RID-10A (refractive index detector) manufactured by Shimadzu Corporation as a detector were used. The type of column used was PLgel 10 um MiniMIX-B manufactured by Agilent Technologies, Inc., the eluent was tetrahydrofuran (manufactured by KANTO CHEMICAL CO., INC., for HPLC), the column temperature was 40°C, and the flow rate was 0.4 ml/min.

<Monomer unit content in chloroprene copolymer>

The content of the component derived from 2-methyl-1,3-butadiene (A-2) in the chloroprene copolymer was determined by $^1$H-NMR analysis. The chloroprene copolymer latex provided was coagulated with methanol. After drying, deuterated chloroform was added to the coagulated product provided. The substance insoluble in deuterated chloroform was filtered off, and the solution provided was subjected to $^1$H-NMR analysis. For the $^1$H-NMR analysis, JNM-AL400 manufactured by JEOL Ltd was used as the measurement apparatus, and tetramethylsilane was used as a reference for the chemical shift.

The content of the component derived from 2-methyl-1,3-butadiene (A-2) was calculated from a peak (5.4 ppm) assigned to 2-chloro-1,3-butadiene (A-1) and a peak (5.1 ppm) assigned to 2-methyl-1,3-butadiene (A-2) in the $^1$H-NMR spectrum by the expression (3).

```
Content of component derived from 2-methyl-1,3-butadiene (A-
2) (mol%)
= (area of peak at 5.1 ppm)/(area of peak at 5.1 ppm + area
of peak at 5.4 ppm) × 100···(3)
```

When the monomer (A-4) is contained but has no peak overlapping the peak at 5.1 ppm and the peak at 5.4 ppm, the expression (3) can be used for determining the proportion of 2-methyl-1,3-butadiene (A-2) based on the sum of 2-chloro-1,3-butadiene (A-1) and 2-methyl-1,3-butadiene (A-2).

When the proportion of the monomer (A-4) contained is determined, the proportion of the monomer (A-4) based on the sum of the 2-chloro-1,3-butadiene (A-1) and the monomer (A-4) is calculated by an expression similar to the expression (3), by use of the peak area of peaks overlapping neither the peaks of 2-chloro-1,3-butadiene (A-1) nor 2-methyl-1,3-butadiene (A-2) among peaks assigned to the monomer (A-4). Similarly, the proportion of the monomer (A-4) also can be determined with respect to 100 mol% in total of the monomer units derived from 2-chloro-1,3-butadiene (A-1) and the monomer units derived from 2-methyl-1,3-butadiene (A-2).

When the monomer (A-4) has peaks overlapping the peak at 5.1 ppm and the peak at 5.4 ppm, the respective peaks assigned to 2-chloro-1,3-butadiene (A-1), 2-methyl-1,3-butadiene (A-2), and the monomer (A-4) are identified using multidimensional NMR measurement results such as $^1$H-$^1$H COSY (COrrelation SpectroscopY), and the peak area can be used for the similar calculation to thereby determine the proportion of each substance.

[Example 1]

(1) Preparation of chloroprene copolymer latex (A)

To a reactor having an internal volume of 5 L, fed were 1200 g of 2-chloro-1,3-butadiene (A-1), 300 g of 2-methyl-1,3-butadiene (A-2), 3.8 g of sulfur (Tsurumi Chemical Industry Co., Ltd., "GOLDEN FLOWER" SULFUR POWDER) (A-3), 1360 g of pure water, 65 g of disproportionated rosin acid (manufactured by Arakawa Chemical Industries, Ltd., R-600), 2.3 g of dimer acid (Harima Chemicals Group, Inc., Haridimer 200), 54.0 g of sodium hydroxide, 21.1 g of a sodium salt of a β-naphthalenesulfonic acid-formalin condensate, and 11.7 mg of copper sulfate. The starting materials fed in the reactor were emulsified, and the rosin acid was converted into rosin acid soap.

2-Chloro-1,3-butadiene (A-1) and 2-methyl-1,3-butadiene (A-2) were blended as material monomers for a chloroprene copolymer, and pure water was blended as a dispersion medium for emulsion polymerization. Rosin acid, dimer acid, and sodium hydroxide were blended as materials for an emulsifier, and the sodium salt of a β-naphthalenesulfonic acid-formalin condensate was blended as an emulsifier. Copper sulfate was blended as a cocatalyst for emulsion polymerization.

To an emulsion obtained by emulsifying the starting materials, 4 g of potassium persulfate was added as a polymerization initiator, and emulsion polymerization was conducted under a nitrogen gas atmosphere at 30°C. When the polymerization conversion of all the monomers reached 84% by mass (7.6 hours later), the polymerization was terminated. A method for calculating the polymerization conversion will be described below. Subsequently, unreacted 2-chloro-1,3-butadiene (A-1) and 2-methyl-1,3-butadiene (A-2) were removed by steam distillation to provide a chloroprene copolymer latex (A1).

(2) Preparation of chloroprene copolymer latex composition

To the chloroprene copolymer latex (A1) provided in (1), zinc oxide (B), a vulcanization accelerator (C), sulfur (D), and an antioxidant (E) were added. The amounts of these added are as follows: per 100 parts by mass of the solid content in the chloroprene copolymer latex (A1), 0.5 parts by mass of zinc oxide (AZ-SW manufactured by Osaki Industry Co., Ltd.), 0.5 parts by mass of zinc dibutyldithiocarbamate (NOCCELER(registered trademark) BZ manufactured by Ouchi Shinko Chemical Industrial Co., Ltd.), 0.5 parts by mass of zinc 2-mercaptobenzothiazole (NOCCELER(registered trademark) MZ manufactured by Ouchi Shinko Chemical Industrial Co., Ltd.), 0.25 parts by mass of diphenyl guanidine (NOCCELER(registered trademark) D manufactured by Ouchi Shinko Chemical Industrial Co., Ltd.), 1.5 parts by mass of sulfur (S-50 manufactured by NIPPON COLOR Ind. Co., Ltd.), and 2.0 parts by mass of a phenolic antioxidant (K-840 manufactured by Chukyo Yushi Co., Ltd.). The chloroprene copolymer latex (A1), zinc oxide (B), vulcanization accelerator (C), sulfur (D), and antioxidant (E) each in the amount described above were fed in a vessel equipped with a stirrer. These components were stirred for 20 minutes and homogeneously mixed to provide a chloroprene copolymer latex composition. The chloroprene copolymer latex composition after stirring was allowed to stand at 23°C for 20 hours for maturing.

The zinc oxide (AZ-SW), sulfur (S-50), and phenolic antioxidant (K-840) were each in the form of a dispersion, which includes the zinc oxide (B), the sulfur (D), or the antioxidant (E) as an active ingredient dispersed in a liquid medium. The amount of each of the above-described zinc oxide (AZ-SW), sulfur (S-50), and phenolic antioxidant (K-840) fed is only the amount of the active ingredient of each of the zinc oxide (AZ-SW), the sulfur (S-50), and the phenolic antioxidant (K-840) fed.

(3) Production of film

The chloroprene copolymer latex composition provided in the above (2) was used to mold a film of the chloroprene copolymer by the dip processing method.

As a mold for a film of the chloroprene copolymer, a ceramic plate of 200 mm in length, 100 mm in width, and 5 mm in thickness was provided. This mold was dipped in a 30% by mass calcium nitrate aqueous solution, then withdrawn, and dried in an oven at 40°C for 10 minutes to thereby cause calcium nitrate, as a coagulant, to adhere to the surface of the mold.

Further, the dried mold was dipped in the chloroprene copolymer latex composition provided in the above (2) to cause the solid content of the chloroprene copolymer latex composition to deposit on the surface of the mold. The mold was withdrawn from the chloroprene copolymer latex composition and then dried in an oven at 70°C for 30 minutes.

Next, the mold with the solid content deposited on the surface thereof was heated in an oven at 110°C for 30 minutes to cure the solid content of the chloroprene copolymer latex composition deposited on the surface of the mold by vulcanization treatment. After left to cool under atmospheric air, the molded article cured on the surface of the mold was cut into a desired shape and size to thereby provide a film as the vulcanized chloroprene copolymer rubber molded article.

(4) Method for measuring physical properties of molded article

The film was cut so as to correspond to the No. 6 dumbbell specified in JIS K6251:2017 to provide a specimen. The specimen has a thickness of 0.15 to 0.25 mm.

<Tensile strength, tensile elongation ratio, and elastic modulus>

A tensile test at 23°C by a method in accordance with JIS K6251-2017 was conducted to measure the tensile strength, the tensile elongation ratio, the elastic modulus at 100% elongation (100% elastic modulus), and the elastic modulus at 500% elongation (500% elastic modulus). The various physical properties of the film measured are summarized in Table 1.

<Elastic modulus retention>

A specimen obtained by the same method as described above was pulled at 23°C at a tensile rate of 200 mm/min until an elongation ratio of 100% and maintained for two minutes. The ratio of the initial stress and the stress after two minutes was determined by the expression (4) and taken as the elastic modulus retention.

```
100% elastic modulus retention (%)

= (elastic modulus at elongation of an elongation ratio of

100% after two minutes)/(initial elastic modulus at

elongation of an elongation ratio of 100%) × 100···(4)
```

The various physical properties of the film measured as described above are summarized in Table 1.

[Examples 2 to 8]

Chloroprene copolymer latex compositions were prepared by the same method as that of Example 1 except that the polymerization conditions and blending conditions were as shown in Table 1. Further, films and specimens were produced and subjected to various evaluations in the same manner as in Example 1. The results are shown in Table 1.

[Comparative Examples 1 to 3]

Comparative Examples 1 to 3 are Comparative Examples of a case in which the sulfur (A-3) is allowed not to coexist in polymerization of the chloroprene copolymer and the sulfur (D) is used in preparation of the latex composition. Chloroprene copolymer latex compositions were prepared by the same method as that of Example 1 except that the polymerization conditions and blending conditions were as shown in Table 2. Further, films and specimens were produced and subjected to various evaluations in the same manner as in Example 1. The results are shown in Table 2.

[Comparative Example 4]

Comparative Example 4 is a Comparative Example of a case in which the sulfur (A-3) is allowed not to coexist but n-dodecylmercaptan is allowed to coexist in polymerization of the chloroprene copolymer. In Comparative Example 4, the chloroprene copolymer was produced by the same method as in Example 1 except that 17.1 g of potassium hydroxide was added, the amount of sodium hydroxide added was changed to 3.9 g, the amount of the sodium salt of a β-naphthalenesulfonic acid-formalin condensate added was changed to 3.3 g, and 1.65 g of n-dodecylmercaptan was added in polymerization. Further, a chloroprene copolymer latex composition, a film, and a specimen were produced by the same method as in Example 1 and subjected to various evaluations in the same manner as in Example 1. The results are shown in Table 2.

[Comparative Examples 5 to 7]

Comparative Examples 5 to 7 are Comparative Examples in a case in which 2,3-dichloro-1,3-butadiene is copolymerized with 2-chloro-1,3-butadiene (A-1) instead of using 2-methyl-1,3-butadiene (A-2) as a monomer unit in polymerization of the chloroprene copolymer. In Comparative Examples 5 and 6, the sulfur (A-3) is allowed not to coexist in copolymerization, and in Comparative Example 7, the sulfur (A-3) is allowed to coexist in copolymerization.

Chloroprene copolymer latex compositions were prepared by the same method as that of Example 1 except that the polymerization conditions and blending conditions were as shown in Table 2. Further, films and specimens were produced and subjected to various evaluations in the same manner as in Example 1. The results are shown in Table 2.

[Table 1]

| | | Example | | | | | | | |
|---|---|---|---|---|---|---|---|---|---|
| | | 1 | 2 | 3 | 4 | 5 | 6 | 7 | 8 |
| Polymerization conditions | 2-Chloro-1,3-butadiene (A-1) (parts by mass) [(mol%)] | 80 [75.5] | 80 [75.5] | 80 [75.5] | 80 [75.5] | 80 [75.5] | 80 [75.5] | 71 [65.3] | 71 [65.3] |
| | 2-Methyl-1,3-butadiene (A-2) (parts by mass) [(mol%)] | 20 [24.5] | 20 [24.5] | 20 [24.5] | 20 [24.5] | 20 [24.5] | 20 [24.5] | 29 [34.7] | 29 [34.7] |
| | 2,3-Dichloro-1,3-butadiene (parts by mass) [(mol%)] | 0 | 0 | 0 | 0 | 0 | 0 | 0 | 0 |
| | Sulfur (A-3) (parts by mass) | 0.25 | 0.25 | 0.50 | 0.50 | 0.10 | 0.10 | 0.25 | 0.25 |
| | n-Dodecylmercaptan (parts by mass) | 0 | 0 | 0 | 0 | 0 | 0 | 0 | 0 |
| | Polymerization time (hours) | 7.6 | 7.6 | 6.8 | 6.8 | 6.0 | 6.0 | 7.7 | 7.7 |
| | Polymerization conversion (% by mass) | 84 | 84 | 82 | 82 | 82 | 82 | 77 | 77 |
| Latex physical properties | Solid content (% by mass) | 41 | 41 | 42 | 42 | 40 | 40 | 41 | 41 |
| | THF insoluble content ratio of copolymer (% by mass) | 14 | 14 | 13 | 13 | 71 | 71 | 40 | 40 |
| | Weight average molecular weight (Mw) of THF soluble component of copolymer ($\times 10^5$) | 4.2 | 4.2 | 6.6 | 6.6 | 6.6 | 6.6 | 6.0 | 6.0 |
| | Proportion of monomer units derived from 2-methyl-1,3-butadiene in copolymer (mol%) | 15 | 15 | 14 | 14 | 10 | 10 | 23 | 23 |
| Blending conditions | Zinc dibutyldithiocarbamate (parts by mass) | 0.5 | 0.5 | 0.5 | 0.5 | 0.5 | 0.5 | 0.5 | 0.5 |
| | Zinc 2-mercaptobenzothiazole (parts by mass) | 0.5 | 0 | 0.5 | 0 | 0.5 | 0 | 0.5 | 0 |
| | Diphenyl guanidine (parts by mass) | 0.25 | 0 | 0.25 | 0 | 0.25 | 0 | 0.25 | 0 |
| | N,N'-Diphenyl thiourea (parts by mass) | 0 | 0 | 0 | 0 | 0 | 0 | 0 | 0 |
| | Zinc oxide (parts by mass) | 0.5 | 0.5 | 0.5 | 0.5 | 0.5 | 0.5 | 0.5 | 0.5 |
| | Sulfur (parts by mass) | 1.5 | 1.5 | 1.5 | 1.5 | 1.5 | 1.5 | 1.5 | 1.5 |
| | Antioxidant (parts by mass) | 2.0 | 2.0 | 2.0 | 2.0 | 2.0 | 2.0 | 2.0 | 2.0 |
| Vulcanization | Vulcanization temperature (°C) | 110 | 110 | 110 | 110 | 110 | 110 | 110 | 110 |
| | Vulcanization time (minutes) | 30 | 30 | 30 | 30 | 30 | 30 | 30 | 30 |

(continued)

| | | Example | | | | | | | |
|---|---|---|---|---|---|---|---|---|---|
| | | 1 | 2 | 3 | 4 | 5 | 6 | 7 | 8 |
| Mechanical properties | 100% Elastic modulus (MPa) | 0.99 | 0.92 | 1.06 | 0.86 | 0.99 | 0.97 | 1.09 | 0.85 |
| | 500% Elastic modulus (MPa) | 1.58 | 1.85 | 1.60 | 1.68 | 1.60 | 2.06 | 1.60 | 1.61 |
| | Tensile strength (MPa) | 26.4 | 22.0 | 31.4 | 24.4 | 21.0 | 20.6 | 27.7 | 20.3 |
| | Tensile elongation ratio (%) | 925 | 1050 | 975 | 1050 | 850 | 1050 | 850 | 1050 |
| | 100% Elastic modulus after two minutes (MPa) | 0.91 | 0.83 | 0.96 | 0.77 | 0.90 | 0.88 | 0.99 | 0.77 |
| | 100% Elastic modulus retention (%) | 92 | 91 | 91 | 90 | 91 | 90 | 91 | 90 |

[Table 2]

| | | Comparative Example | | | | | | |
|---|---|---|---|---|---|---|---|---|
| | | 1 | 2 | 3 | 4 | 5 | 6 | 7 |
| Polymerization conditions | 2-Chloro-1,3-butadiene (A-1) (parts by mass) [(mol%)] | 80 [75.5] | 80 [75.5] | 80 [75.5] | 80 [75.5] | 91.5 [95.1] | 91.5 [95.1] | 88 [93.0] |
| | 2-Methyl-1,3-butadiene (A-2) (parts by mass) [(mol%)] | 20 [24.5] | 20 [24.5] | 20 [24.5] | 20 [24.5] | 0 | 0 | 0 |
| | 2,3-Dichloro-1,3-butadiene (parts by mass) [(mol%)] | 0 | 0 | 0 | 0 | 8.5 [4.9] | 8.5 [4.9] | 12 [7.0] |
| | Sulfur (A-3) (parts by mass) | 0 | 0 | 0 | 0 | 0 | 0 | 0.25 |
| | n-Dodecylmercaptan (parts by mass) | 0 | 0 | 0 | 0.11 | 0 | 0 | 0 |
| | Polymerization time (hours) | 6.7 | 6.7 | 6.7 | 6.1 | 5.0 | 5.0 | 5.5 |
| | Polymerization conversion (% by mass) | 81 | 81 | 81 | 84 | 95 | 95 | 94 |
| Latex physical properties | Solid content (% by mass) | 43 | 43 | 43 | 45 | 51 | 51 | 46 |
| | THF insoluble content ratio of copolymer (% by mass) | 75 | 75 | 75 | 2 | 41 | 41 | 35 |
| | Weight average molecular weight (Mw) of THF soluble component of copolymer ($\times 10^5$) | 4.0 | 4.0 | 4.0 | 6.0 | 4.9 | 4.9 | 5.0 |
| | Proportion of monomer units derived from 2-methyl-1,3-butadiene in copolymer (mol%) | 17 | 17 | 17 | 14 | 0 | 0 | 0 |
| Blending conditions | Zinc dibutyldithiocarbamate (parts by mass) | 0.5 | 0.5 | 0.5 | 0.5 | 0 | 0.5 | 0.5 |
| | Zinc 2-mercaptobenzothiazole (parts by mass) | 0.5 | 0 | 0.5 | 0.5 | 0 | 0.5 | 0 |
| | Diphenyl guanidine (parts by mass) | 0.25 | 0 | 0.25 | 0.25 | 1.0 | 0 | 0 |
| | N,N'-Diphenyl thiourea (parts by mass) | 0 | 0 | 0 | 0 | 1.0 | 0 | 0 |
| | Zinc oxide (parts by mass) | 0.5 | 0.5 | 0.5 | 0.5 | 5.0 | 0.5 | 5.0 |
| | Sulfur (parts by mass) | 1.5 | 1.5 | 3.0 | 1.5 | 0 | 1.5 | 0 |
| | Antioxidant (parts by mass) | 2.0 | 2.0 | 2.0 | 2.0 | 2.0 | 2.0 | 2.0 |
| Vulcanization | Vulcanization temperature (°C) | 110 | 110 | 110 | 110 | 130 | 130 | 110 |
| | Vulcanization time (minutes) | 30 | 30 | 30 | 30 | 30 | 30 | 30 |

(continued)

| | | Comparative Example | | | | | | |
| | | 1 | 2 | 3 | 4 | 5 | 6 | 7 |
| Mechanical properties | 100% Elastic modulus (MPa) | 0.89 | 0.72 | 0.76 | 0.63 | 0.79 | 0.58 | 0.88 |
| | 500% Elastic modulus (MPa) | 2.20 | 1.64 | 2.00 | 1.28 | 2.12 | 1.11 | 2.16 |
| | Tensile strength (MPa) | 14.0 | 13.3 | 12.5 | 18.9 | 19.7 | 16.9 | 16.4 |
| | Tensile elongation ratio (%) | 1000 | 1000 | 900 | 1150 | 1000 | 1280 | 1010 |
| | 100% Elastic modulus after two minutes (MPa) | 0.77 | 0.61 | 0.62 | 0.54 | 0.65 | 0.41 | 0.68 |
| | 100% Elastic modulus retention (%) | 86 | 85 | 89 | 85 | 82 | 71 | 78 |

Chloroprene copolymer rubber molded articles obtained by vulcanization treatment under mild conditions using the chloroprene copolymer latexes, provided in Examples 1 to 8 by emulsion polymerizing 2-chloro-1,3-butadiene (A-1) and 2-methyl-1,3-butadiene (A-2) in the presence of sulfur (A-3), have a high tensile strength of 20 MPa or more. Additionally, the chloroprene copolymer rubber molded articles provided in Examples 1 to 8 have a high 100% elastic modulus retention of 90% and small stress relaxation properties. For this reason, gloves produced using the chloroprene copolymer rubber molded articles provided in Examples 1 to 8 are preferred as medical disposable gloves because breaks are unlikely to occur, the followability to extension and contraction generated by motions of hands of a user (flexibility) is high, and the tactile sensations become favorable. Such molded articles are presumed to have been obtained because formation of a crosslinked structure among the chloroprene copolymers present in one particulate of the chloroprene copolymer latex (A) is facilitated by the sulfur (A-3) and further, formation of a crosslinked structure among the chloroprene copolymers present in different particulates of the chloroprene copolymer latex (A) is facilitated by the sulfur (D).

Meanwhile, in Comparative Examples 1 to 4, where copolymerization was conducted in the absence of the sulfur (A-3), the tensile strength and 100% elastic modulus retention have not reached the performance required for materials for medical disposable gloves. When the amount of the sulfur (D) to be blended in the latex composition is increased as in Comparative Example 3, the 100% elastic modulus retention is improved but the tensile strength will be a lower value in comparison with the mechanical properties shown in Examples 1 to 4. In Comparative Examples 1 to 4, unlike in Examples 1 to 4, it is presumed that no crosslinked structure of the chloroprene copolymer ascribed to the sulfur (A-3) has been formed and only a crosslinked structure among the chloroprene copolymers due to the sulfur (D) has been formed. It can be presumed that the above results indicated that, when mild vulcanization conditions such as heating at 110°C for 30 minutes are employed, a molded article having performance required for materials for medical disposable gloves cannot be provided only with the crosslinked structure among the chloroprene copolymers ascribed to the sulfur (D), which is blended in Comparative Examples 1 to 4.

The comparative results as above reveals that coexistence of the sulfur (A-3) in emulsion polymerization of the chloroprene copolymer is useful to produce a chloroprene copolymer latex for providing a molded article having flexibility and stress relaxation properties desirable for use in gloves.

As shown in Examples 2, 4, 6, and 8, allowing the sulfur (A-3) to coexist in emulsion polymerization of the chloroprene copolymer results in a tensile strength of 20 MPa or more and a 100% elastic modulus retention of 90% or more, even when the amount of vulcanization accelerator (C) blended is reduced, and a molded article suitably usable as medical disposable gloves can be obtained.

The chloroprene copolymer latex compositions produced in Examples 2, 4, 6, and 8 contain neither diphenyl guanidine nor N,N'-diphenyl thiourea, which has become recognized as a sensitizer in Europe or the like, and thus is more effective for measures for allergy.

As shown in Comparative Examples 5 to 7, when the chloroprene copolymer included in the latex composition does not contain monomer units derived from 2-methyl-1,3-butadiene (A-2), the resulting molded article will have a lower tensile strength and a lower 100% elastic modulus retention.

**Claims**

1. A method for producing a chloroprene copolymer latex (A), comprising a step of emulsion polymerizing 2-chloro-1,3-butadiene (A-1) and 2-methyl-1,3-butadiene (A-2) in the presence of sulfur (A-3).

2. The method for producing a chloroprene copolymer latex (A) according to claim 1, wherein a proportion of 2-methyl-1,3-butadiene (A-2) in the total monomer components used in the step of emulsion polymerization is 2 to 60 mol%, and a polymerization conversion of the total monomers is 61 to 90% by mass.

3. The method for producing a chloroprene copolymer latex (A) according to claim 1 or claim 2, wherein an amount of the sulfur (A-3) to be used in the emulsion polymerization is 0.1 to 1.0 parts by mass per 100 parts by mass of the total amount of 2-chloro-1,3-butadiene (A-1) and 2-methyl-1,3-butadiene (A-2).

4. A chloroprene copolymer latex (A), being a latex of a chloroprene copolymer, and
obtained by emulsion polymerizing 2-chloro-1,3-butadiene (A-1) and 2-methyl-1,3-butadiene (A-2) in the presence of sulfur (A-3).

5. The chloroprene copolymer latex (A) according to claim 4, wherein a tetrahydrofuran insoluble content ratio of the chloroprene copolymer at 25°C is 5 to 80% by mass.

6. The chloroprene copolymer latex (A) according to claim 4 or 5, wherein a tetrahydrofuran soluble component in the chloroprene copolymer at 25°C has a weight average molecular weight of 400,000 or more.

7. The chloroprene copolymer latex (A) according to any one of claims 4 to 6, wherein an amount of the sulfur (A-3) to be used in the emulsion polymerization is 0.1 to 1.0 parts by mass per 100 parts by mass of the total amount of 2-chloro-1,3-butadiene (A-1) and 2-methyl-1,3-butadiene (A-2).

8. The chloroprene copolymer latex (A) according to any one of claims 4 to 7, wherein a proportion of a monomer unit derived from 2-methyl-1,3-butadiene (A-2) is 10 to 50 mol% in the total monomer units constituting the chloroprene copolymer.

9. A chloroprene copolymer latex composition, comprising 100 parts by mass in total of the chloroprene copolymer latex (A) according to any one of claims 4 to 8 and optionally a synthetic rubber (F), and

comprising 0.1 to 20.0 parts by mass of a metal oxide (B),
0.1 to 10.0 parts by mass of a vulcanization accelerator (C),
0.1 to 10.0 parts by mass of sulfur (D), and
0.1 to 10.0 parts by mass of an antioxidant (E).

10. The chloroprene copolymer latex composition according to claim 9, wherein the composition comprises a synthetic rubber(F), and
a proportion of the synthetic rubber (F) is 1 to 50% by mass per 100% by mass in total of solid content of the chloroprene copolymer latex (A) and the synthetic rubber (F).

11. A molded article of a chloroprene copolymer rubber, obtained by curing the chloroprene copolymer latex composition according to claim 9 or 10.

12. A dip-molded product obtained by molding the chloroprene copolymer latex composition according to claim 9 or 10 by a dipping method, followed by curing.

13. The dip-molded product according to claim 12, wherein the dip-molded product is gloves.

14. The dip-molded product according to claim 13, wherein the dip-molded product is medical disposable gloves.

## INTERNATIONAL SEARCH REPORT

International application No.

**PCT/JP2021/041179**

### A. CLASSIFICATION OF SUBJECT MATTER

***C08F 2/44***(2006.01)i; ***A41D 19/00***(2006.01)i; ***A41D 19/04***(2006.01)i; ***A61B 42/10***(2016.01)i; ***B29C 41/14***(2006.01)i;
***C08F 2/22***(2006.01)i; ***C08F 2/38***(2006.01)i; ***C08F 236/18***(2006.01)i; ***C08K 3/06***(2006.01)i; ***C08K 3/22***(2006.01)i;
***C08L 11/02***(2006.01)i
FI:   C08F2/44 A; A41D19/00 P; A41D19/04 B; A61B42/10; B29C41/14; C08F2/22; C08F2/38; C08F236/18; C08K3/06;
C08K3/22; C08L11/02

According to International Patent Classification (IPC) or to both national classification and IPC

### B. FIELDS SEARCHED

Minimum documentation searched (classification system followed by classification symbols)

C08F2/00-299/08; C08L1/00-101/16; C08K3/00-13/08; A41D19/00-19/04; A61B42/00-42/60; B29C41/00-41/52

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2003-55409 A (DENKI KAGAKU KOGYO KK) 26 February 2003 (2003-02-26) claims, paragraphs [0007], [0024], column "example", etc. | 1-14 |
| X | SU 479369 A (KARAPETYAN, N. G. et al.) 23 October 1980 (1980-10-23) claims, column "examples", etc. | 1-14 |
| X | SU 393284 A (ARZUMANYAN, M. N. et al.) 10 August 1973 (1973-08-10) claims, column "examples", etc. | 1-14 |
| A | WO 2020/065789 A1 (SHOWA DENKO KK) 02 April 2020 (2020-04-02) | 1-14 |
| A | JP 2019-44116 A (SHOWA DENKO KK) 22 March 2019 (2019-03-22) | 1-14 |
| P, A | WO 2021/079981 A1 (SHOWA DENKO KK) 29 April 2021 (2021-04-29) | 1-14 |
| P, A | WO 2021/132460 A1 (SHOWA DENKO KK) 01 July 2021 (2021-07-01) | 1-14 |

☐ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 January 2022** | **25 January 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

| International application No. |
| --- |
| **PCT/JP2021/041179** |

| Patent document cited in search report | | | Publication date (day/month/year) | Patent family member(s) | | | Publication date (day/month/year) |
| --- | --- | --- | --- | --- | --- | --- | --- |
| JP | 2003-55409 | A | 26 February 2003 | (Family: none) | | | |
| SU | 479369 | A | 23 October 1980 | (Family: none) | | | |
| SU | 393284 | A | 10 August 1973 | (Family: none) | | | |
| WO | 2020/065789 | A1 | 02 April 2020 | CN | 112673063 | A | |
| JP | 2019-44116 | A | 22 March 2019 | (Family: none) | | | |
| WO | 2021/079981 | A1 | 29 April 2021 | (Family: none) | | | |
| WO | 2021/132460 | A1 | 01 July 2021 | (Family: none) | | | |

Form PCT/ISA/210 (patent family annex) (January 2015)

**EP 4 245 778 A1**

**Patent documents cited in the description**

- JP 2019143002 A
- JP 2019044116 A
- JP 2001131812 A